(19) Europäisches Patentamt European Patent Office Office européen des brevets

(11) **EP 4 098 270 B1**

(12) **EUROPEAN PATENT SPECIFICATION**

(45) Date of publication and mention of the grant of the patent:
**29.01.2025 Bulletin 2025/05**

(21) Application number: **22175710.7**

(22) Date of filing: **27.05.2022**

(51) International Patent Classification (IPC):
**A61K 35/50** (2015.01)     **A61P 27/02** (2006.01)

(52) Cooperative Patent Classification (CPC):
**A61K 35/50; A61P 27/02**

(54) **USE OF SECRETOME OF AMNIOTIC FLUID STEM CELLS IN THE TREATMENT OF DRY EYE DISEASE**

VERWENDUNG DES SEKRETOMS VON STAMMZELLEN AUS FRUCHTWASSER BEI DER BEHANDLUNG DES TROCKENEN AUGES

UTILISATION DU SÉCRÉTOME DES CELLULES SOUCHES DU LIQUIDE AMNIOTIQUE DANS LE TRAITEMENT DE LA MALADIE DE L' IL SEC

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR

(30) Priority: **01.06.2021 US 202117335477**

(43) Date of publication of application:
**07.12.2022 Bulletin 2022/49**

(73) Proprietor: **U-Neuron Biomedical Inc.**
**New Taipei City 221 (TW)**

(72) Inventors:
• **Hwang, Shiaw-Min**
**221 New Taipei City (TW)**
• **Hsieh, Chen-An**
**221 New Taipei City (TW)**
• **Lin, Pei-Cheng**
**221 New Taipei City (TW)**

(74) Representative: **Straus, Alexander**
**2K Patent- und Rechtsanwälte**
**Bajuwarenring 14**
**82041 Oberhaching (DE)**

(56) References cited:
US-A1- 2008 286 378     US-A1- 2017 100 440
US-A1- 2018 221 417

• HIGA KAZUNARI ET AL: "Effects of Amniotic Membrane-Derived Fibroblast Supernatant on Corneal Epithelium", INVESTIGATIVE OPTHALMOLOGY & VISUAL SCIENCE, vol. 60, no. 12, 3 September 2019 (2019-09-03), US, pages 3718, XP055952982, ISSN: 1552-5783, DOI: 10.1167/iovs.19-27041
• COSTA AMBRA ET AL: "Comprehensive Profiling of Secretome Formulations from Fetal- and Perinatal Human Amniotic Fluid Stem Cells", INTERNATIONAL JOURNAL OF MOLECULAR SCIENCES, vol. 22, no. 7, 2 April 2021 (2021-04-02), pages 3713, XP055953066, DOI: 10.3390/ijms22073713
• CUI XINHAN ET AL: "Assessment of Corneal Epithelial Thickness in Dry Eye Patients", OPTOMETRY AND VISION SCIENCE., vol. 91, no. 12, 1 December 2014 (2014-12-01), US, pages 1446 - 1454, XP055953330, ISSN: 1040-5488, Retrieved from the Internet <URL:https://www.ncbi.nlm.nih.gov/pmc/articles/PMC4302058/pdf/nihms641360.pdf> DOI: 10.1097/OPX.0000000000000417
• FATHI IBRAHIM ET AL: "Human Amniotic Epithelial Cells Secretome: Components, Bioactivity, and Challenges", FRONTIERS IN MEDICINE, vol. 8, 10 January 2022 (2022-01-10), XP055953372, DOI: 10.3389/fmed.2021.763141

EP 4 098 270 B1

**(Cont. next page)**

- **SIMONI GIUSEPPE ET AL: "The amniotic fluid-derived cells: the biomedical challenge for the third millennium", JOURNAL OF PRENATAL MEDICINE, 1 July 2009 (2009-07-01), Italy, pages 34 - 36, XP055953380, Retrieved from the Internet <URL:https://www.ncbi.nlm.nih.gov/pmc/articles/PMC3279105/pdf/prenatal-03-0034.pdf> [retrieved on 20220822]**

**Description**

FIELD OF THE INVENTION

The present disclosure is related to use of a secretome of amniotic fluid stem cells in treating a dry eye disease and in treating a cornea wound.

BACKGROUND OF THE INVENTION

Amniotic fluid cells are used as a routine prenatal diagnosis to detect whether the fetal chromosomes are abnormal. In addition, it was found that some small nucleated cells with the characteristics of hematopoietic progenitor cells can be identified in the amniotic fluid before 12 weeks of pregnancy, suggesting that these cells may come from yolk sac. There were some reports from 2004 to 2006 of successful isolation and identification of existence of another group of stem cells in amniotic fluid. These amniotic fluid stem cells were proved to express specific biomarkers of both mesenchymal stem cells and neural stem cells, with the ability of differentiating into bones, cartilage, fat, and nerve cells. It is confirmed that amniotic fluid stem cells can proliferate stably in large quantities in vitro, and they proliferate faster than mesenchymal stem cells obtained from adult bone marrow and fat, without the tumorigenic potential of embryonic stem cells. Therefore, amniotic fluid stem cells have the potential of application to clinical medicine.

Dry eye disease is generally known as keratoconjunctivitis sicca. Millions of people are suffering from this condition every year, especially due to the rapid development of electronic products which is the most common reason for causing dry eye disease nowadays. Dry eye symptoms have traditionally been managed with eyelid hygiene, topical antibiotics, tetracyclines, doxycycline, anti-inflammatory compounds (cyclosporine), or corticosteroids which are often time consuming, frustrating, and frequently ineffective or variably effective treatments.

Accordingly, there is a need to develop a new therapeutic agent for the dry eye disease that is capable to effectively alleviate the conditions.

US2017100440 A1 discloses a human amniotic fluid formulation for topical application to the eye, for treating ocular diseases and injuries including dry eyes, Sjogren's Syndrome, cataracts, burns and injuries to the eye tissues. The formulation is a sterile de-cellularized human amniotic fluid (D-HAF), devoid of amniotic stem cells and elements of micronized membrane or chorion particles.

US2008286378 A1 discloses a composition for the treatment of ocular disease and injury for administration of amniotic fluid directly to the eye, the fluid being free of amniotic membrane particulate matter for example. The indications include chemical burns, dry eye and corneal neovascular disorders, corneal opacities (e.g. corneal haze) and inflammatory diseases of the eye.

SUMMARY OF THE INVENTION

References to methods of treatment are to be interpreted as pharmaceutical compositions for use in the respective method.

In one aspect, the present invention provides a pharmaceutical composition for use in a method for treating a dry eye disease in a subject, the method comprising administering to said subject said composition comprising a secretome of amniotic fluid stem cells and a pharmaceutically acceptable carrier; wherein the secretome is prepared by a method comprising the following steps: culturing amniotic fluid stem cells in a basal medium for 24-72 hours to obtain a (conditioned) culture medium; collecting a supernatant of the culture medium after centrifugation, and filtrating the supernatant to obtain the secretome.

In one embodiment, the supernatant is filtrated by a filter having a pore size less than 0.5 $\mu$m; preferably, the pore size is less than 0.22 $\mu$m.

According to certain embodiments of the present invention, the composition is administered topically to the subject's eye, preferably in a form of eye gel, eye drop, eye bath solution, eye lotion, eye insert, eye ointment, or eye spray.

In another aspect, a method for corneal epithelia wound healing in a subject is provided, comprising administering to said subject a composition comprising a therapeutically effective amount of a secretome of amniotic fluid stem cells as disclosed herein, and a pharmaceutically acceptable carrier.

In one further aspect, the present invention provides a pharmaceutical composition for use in treating a dry eye disease. The pharmaceutical composition comprises a secretome of amniotic fluid stem cells, wherein the secretome is prepared by a method comprising the following steps: culturing amniotic fluid stem cells in a basal medium for 24-72 hours to obtain a (conditioned) culture medium, collecting a supernatant of the culture medium after centrifugation, and filtrating the supernatant to obtain the secretome. The pharmaceutical composition may further comprise a pharmaceutically acceptable carrier.

The present invention will be further described by way of the following examples. However, it should be understood that the following examples are solely intended for the purpose of illustration and should not be construed as limiting the disclosure in practice.

BRIEF DESCRIPTION OF THE DRAWINGS

The foregoing summary, as well as the following detailed description of the invention, will be better understood when read in conjunction with the appended drawings. For the purpose of illustrating the invention, there are shown in the drawings embodiments which are presently preferred.

In the drawings:

Fig. 1 shows the schematic timeline of experiment of Example 1.
Fig. 2 shows the profiles of tear volume collected from each of mice in Example 1 on Day 0, Day 4, and Day 7 according to Fig. 1 (*p<0.05, compared with Damage group).
Fig. 3 shows the profiles of tear break up time (TBUT) measured on the eyes of each mouse in Example 1 on Day 0, Day 4, and Day 7 according to Fig. 1 (*p<0.05, compared with Damage group).
Fig. 4 shows the corneal surface photography of the mice in Example 1 which are subject to UVB damage on eyes, including (A) the assessments of cornea opacity, (B) cornea smoothness, (C) cornea topography, and (D) Lissamine Green Stain examination; wherein the higher score of assessment represents the higher severity of damage (*p<0.05, compared with Damage group).
Fig. 5 shows the result of MTT assay illustrating the correlation of treatment of different concentrations of secretome of AFSC and the alive cell number of human corneal epithelial cells at 24, 48, and 72 hours in Example 2 (*p<0.05, compared with concentration 0).
Fig. 6 shows the result of wound healing assay illustrating the efficiency of migration of human corneal epithelial cells upon the treatment of secretome of AFSC at 6, 12, 24, and 48 hours (*p<0.05, **p<0.001, compared with concentration 0).

DESCRIPTION OF THE INVENTION

The following embodiments when read with the accompanying drawings are made to clearly exhibit the above-mentioned and other technical contents, features and effects of the present disclosure. Through the description by means of the embodiments, a person of ordinary skills in the art would explicitly understand the technical approach and effects the present disclosure adopts to achieve the above-identified aspect.

Unless otherwise defined, all the technical and scientific terms used herein have the same definition as commonly understood by a person of ordinary skills in the art to which the present disclosure pertains.

As used in the specification and the appended claims, the singular forms "a," "an" and "the" include plural referents unless the context clearly dictates otherwise. In this application, the use of "or" or "and" means "and/or" unless stated otherwise. Furthermore, use of the term "including" as well as other forms, such as "include", "includes," and "included," is not limiting. The section headings used herein are for organizational purposes only and are not to be construed as limiting the subject matter described. Unless otherwise specified, all the material used herein is commercial and can be easily obtained.

As used herein, the term "about" or the like used herein refers to a measured quantity, such as dose, including the deviation $\pm 15\%$ or $\pm 10\%$ relative to a specified quantity in an embodiment; the deviation $\pm 5\%$ relative to a specified quantity in a preferred embodiment; the deviation $\pm 1\%$ relative to a specified quantity in a further preferred embodiment; or the deviation $\pm 0.1\%$ relative to a specified quantity in a most preferred embodiment; whereas the nature of the substance the quantity pertains to is not affected thereby.

As used herein, the term "disease" used herein refers to any condition, infection, disorder, or syndrome that requires medical intervention or for which medical intervention is desirable. Such medical intervention can include treatment, diagnosis, and/or prevention.

The terms "dry eye disease (DED)," "dry eye syndrome (DES)," "keratoconjunctivitis sicca (KCS)," or simply "dry eyes" are used interchangeably to refer to the eye disease caused by decreased tear production or increased tear film evaporation. Dry eye disease may be as a result of another underlying condition causing dry eye, for example, Sjogren's syndrome, menopause or rheumatoid arthritis. Dry eye may also be one of the complications of inflammation. Dry eye may also be the result of infection, or a side effect of medications, or exposure to toxins, chemicals, or other substances may cause a symptom or condition of dry eye. Dry eye disease may be manifested by one or more ophthalmologic clinical symptoms as known in the art, including but not limited to dryness, foreign body sensation, burning, itching, irritation, redness, eye pain, blurred vision and/or degraded vision.

As used herein, the term "secretome," also known as "conditioned medium," refers to the totality (or collection) of proteins secreted from cells to the environment of cells (into culture medium) when the cells are cultured.

As used herein, the term "stem cells" is a generic term for undifferentiated cells before differentiation into respective cells constituting tissues, and the stem cells have an ability to differentiate into particular cells by particular differentiation stimulations. According to the present disclosure, the cells used in the preparation of the secretome of the present disclosure are the amniotic fluid stem cells.

The term "composition" as used herein, refers a product that results from the mixing or combining of more than one active ingredient and includes both fixed and non-fixed combinations of the active ingredients. The term "fixed combination" means that the active ingredients and a certain co-agent are both administered to a patient simultaneously in the form of a single entity or dosage. The term "non-fixed combination" means that the active ingredients and a certain co-agent are administered to a patient as separate entities either simultaneously, concurrently or sequentially with no specific intervening time limits, wherein such administration provides effective levels of the two agents in the body of the patient. The latter also applies to cocktail therapy, e.g. the administration of three or more active ingredients.

According to the present invention, the term "pharmaceutical composition" used herein refers to a therapeutically effective amount of secretome or conditioned medium of amniotic fluid stem cells and optionally a pharmaceutically acceptable carrier.

The term "pharmaceutically acceptable" used herein refers to the situation in which within the scope of reasonable medical judgment, a drug is suitable for use in contact with a tissue of a subject (such as a human) taking the drug, without excessive toxicity, irritation, allergic reaction, or other problems or complications, and with reasonable benefit/risk ratio. Each carrier must be compatible with other ingredients in order to be "acceptable".

The term "carrier" used herein refers to a non-toxic compound or agent that has the function of assisting cells or tissues to absorb active ingredient. The carrier is selected from, for example, excipients, adjuvants, diluents, fillers, or bulking agents, granulating agents, coating agents, release control agents, binding agents, disintegrants, lubricants, preservatives, surfactants, antioxidants, buffers, suspending agents, thickeners, stabilizers, or other carriers used in pharmaceutical compositions. Examples of carriers include, but are not limited to, polyvinyl alcohol, povidone, hydroxypropyl methyl cellulose, poloxamers, hydroxyethyl cellulose cyclodextrin, carboxymethylcellulose (CMC), phosphate buffered saline (PBS), water, emulsifier (such as oil and water emulsifier), or wetting agent. Tonicity adjustors may be added as needed or convenient. They include, but are not limited to, salts, particularly sodium chloride, potassium chloride, mannitol and glycerin, or any other suitable ophthalmically acceptable tonicity adjustor. Various buffers and means for adjusting pH may be used so long as the resulting preparation is ophthalmically acceptable. Accordingly, buffers include acetate buffers, citrate buffers, phosphate buffers and borate buffers. Acids or bases may be used to adjust the pH of these formulations as needed. Similarly, an ophthalmically acceptable antioxidant for use in the present disclosure includes, but is not limited to, sodium metabisulfite, sodium thiosulfate, acetylcysteine, butylated hydroxyanisole and butylated hydroxytoluene. Other excipient components which may be included in the ophthalmic preparations are chelating agents and antibiotics. The preferred chelating agent is edetate disodium, although other chelating agents may also be used in place of or in conjunction with it. Non-limiting examples of antibiotics useful in the present disclosure include trimethoprim sulfate/polymyxin B sulfate, gatifloxacin, moxifloxacin hydrochloride, tobramycin, teicoplanin, vancomycin, azithromycin, clarithromycin, amoxicillin, penicillin, ampicillin, carbenicillin, ciprofloxacin, levofloxacin, amikacin, gentamicin, kanamycin, neomycin and streptomycin,

The terms "effective amount" or "therapeutically effective amount," as used herein, refer to a sufficient amount of an agent or a compound being administered which will relieve to some extent one or more of the symptoms of the disease or condition being treated. The result can be reduction and/or alleviation of the signs, symptoms, or causes of a disease, or any other desired alteration of a biological system. For example, an "effective amount" for therapeutic uses is the amount of the composition comprising a peptide or protein as disclosed herein required to provide a clinically significant decrease in disease symptoms. An appropriate "effective" amount in any individual case may be determined using techniques, such as a dose escalation study.

The terms "treat," "treating" or "treatment," as used herein, include alleviating, abating or ameliorating at least one symptom of a disease or condition, preventing additional symptoms, inhibiting the disease or condition, e.g., arresting the development of the disease or condition, relieving the disease or condition, causing regression of the disease or condition, relieving a condition caused by the disease or condition, or stopping the symptoms of the disease or condition either prophylactically and/or therapeutically.

The present invention provides a composition for use in the treatment of a dry eye disease or treatment of a corneal epithelia wound, comprising a secretome of amniotic fluid stem cells, wherein the secretome is prepared by a method comprising the following steps: incubating amniotic fluid stem cells in a basal medium for 24-72 hours; collecting and centrifuging a supernatant of the basal medium; and filtrating the supernatant to obtain the secretome.

In one embodiment, the supernatant is filtrated by a filter having a pore size less than 0.5 $\mu$m. Preferably, the pore size of the filter is 0.45 $\mu$m, 0.3 $\mu$m, or 0.22 $\mu$m.

In one embodiment, the composition is a pharmaceutical composition further comprising a pharmaceutically acceptable carrier.

Disclosed, but not part of the invention, are the following methods:

A method for treating a dry eye disease comprises administering to a subject in need thereof a pharmaceutical composition comprising secretome of amniotic fluid stem cells.

A method for corneal epithelia wound healing comprises administering to a subject in need thereof a pharmaceutical composition comprising secretome of amniotic fluid stem cells.

The pharmaceutical composition may be administered topically to the subject's eye. In other words, the pharmaceutical composition may be formulated for use in topical administration.

According to certain embodiments of the present invention, the pharmaceutical composition is in a form of ophthalmological formulation, such as eye gel, eye drop, eye bath solution, eye lotion, eye insert, eye ointment, or eye spray.

The present invention is further illustrated by the following examples, which are provided for the purpose of demonstration rather than limitation.

**Examples**

**Example 1**

The therapeutic AFSC were obtained by a method comprising the steps of thawing cells from qualified cell banks, such as the working cell bank (WCB) and culturing the cells in MSC NutriStem® XF medium (serum free/xeno free). The AFSCs were further incubated in basal medium (alpha-minimum essential medium) for 48 hours to collect the conditioned medium of AFSC.

Subsequently, the conditioned medium collected from cell culture was centrifuged in a speed of 2,000 rpm for about 10 minutes and filtrated by a 0.22 $\mu$m filter to remove cell residues, and then the secretome of AFSC was obtained, which was stored in -20°C refrigerator for further use.

**Example 2**

In the present example, the animal model of dry eye was induced via UVB irradiation, and the efficacy of secretome of amniotic fluid stem cells in the treatment of dry eye was assessed based on the result of experiment.

**Materials and Method**

Female ICR albino mice (BioLASCO) were used as animal model in the present example. The experiment began when the mice are 5 to 6 weeks old. The ICR mice were divided into 6 groups, each of which includes 6 mice (n=6):

1. Health control (Blank): treated with 0.9% saline solution by eye-drop;
2. Positive control (Damage): treated with 0.9% saline solution by eye-drop and UVB to cause damage;
3. 1/5 Secretome of amniotic fluid stem cells (1/5D): treated with UVB to cause damage and also with 1/5 dilution of secretome of amniotic fluid stem cells (AFSC);
4. 1/5 Secretome of amniotic fluid stem cells (1/5D): treated with UVB to cause damage and also with 1/5 dilution of secretome of AFSC by eye-drop;
5. 1/10 Secretome of amniotic fluid stem cells (1/10D): treated with UVB to cause damage and also with 1/10 dilution of secretome of AFSC by eye-drop;
6. 1/20 Secretome of amniotic fluid stem cells (1/20D): treated with UVB to cause damage and also with 1/20 dilution of secretome of AFSC by eye-drop;
7. Artificial tear control (AFT): treated with 0.9% artificial tears (ALLERGAN) by eye-drop and UVB to cause damage;

All mice were administered with eye drop according to the protocol set forth twice per day at 10:00 and 17:00 since 3 days before the beginning of induction of dry eye. The UVB irradiation was conducted from Day 1 to Day 7 consecutively, wherein the intensity of UVB was 0.72 J/cm$^2$ .

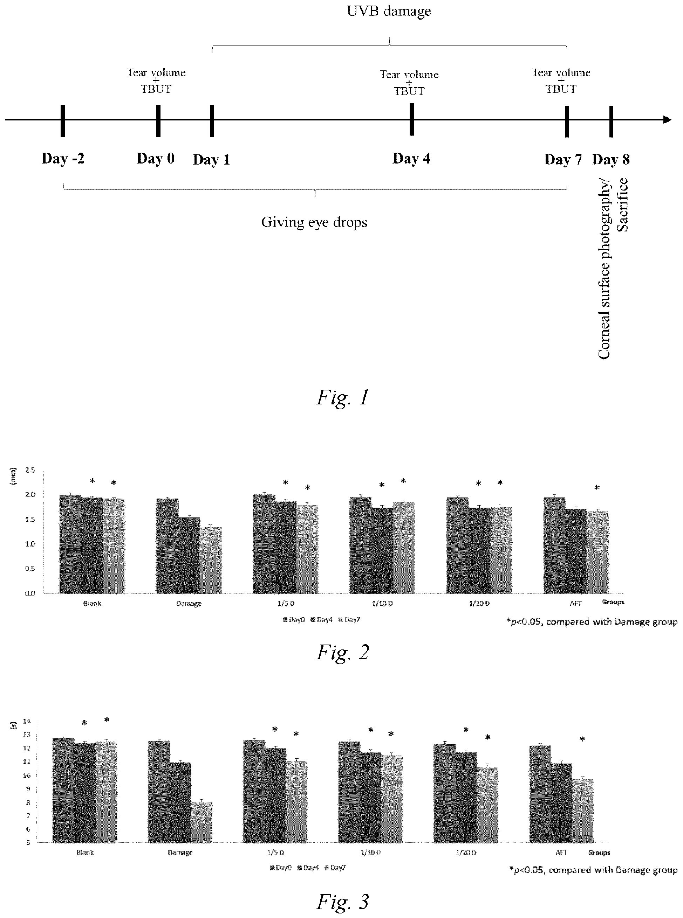

The schematic timeline of experiment is illustrated in Fig. 1. The tear was collected on day 0, 4, and 7 to measure the change of tear volume; the tear break up time (TBUT) was also measured on each mice on day 0, 4, and 7.

The mice were sacrificed on day 8 and further were subject to histopathological analysis.

**Results**

The tear volumes collected from each of mice were measure and the result was shown in Fig. 2. As shown in Fig. 2, the efficacy of secretion of tear was improved in the groups treated with the secretome of AFSC, especially in the group treated with 1/10D at Day 7. The improvement of the groups treated with the secretome of AFSC (at 1/5D, 1/10D, and 1/20D) at Day 4 and Day 7 showed statistically significant as compared with the positive control (Damage) (p<0.05). In contrast, although the mice treated with artificial tear (AFT) showed significant improvement at Day 7, the artificial tears still showed low efficacy in the improvement of secretion of tear as compared to the groups treated with the secretome of AFSC.

The tear break up time (TBUT) was measured on each mice and the result was shown in Fig. 3. As shown in Fig. 3, the TBUT of each group was gradually decreased till Day 7 except the health control. However, the TBUT was improved in the groups treated with the secretome of AFSC (at 1/5D, 1/10D, and 1/20D) at Day 4 and Day 7, which showed statistically significant as compared to the positive control (p<0.05). Although the result did not obviously demonstrate a dose-dependent effect, the efficacy to maintained the stability of tear film was higher in the groups treated with secretome of AFSC compared to the AFT group.

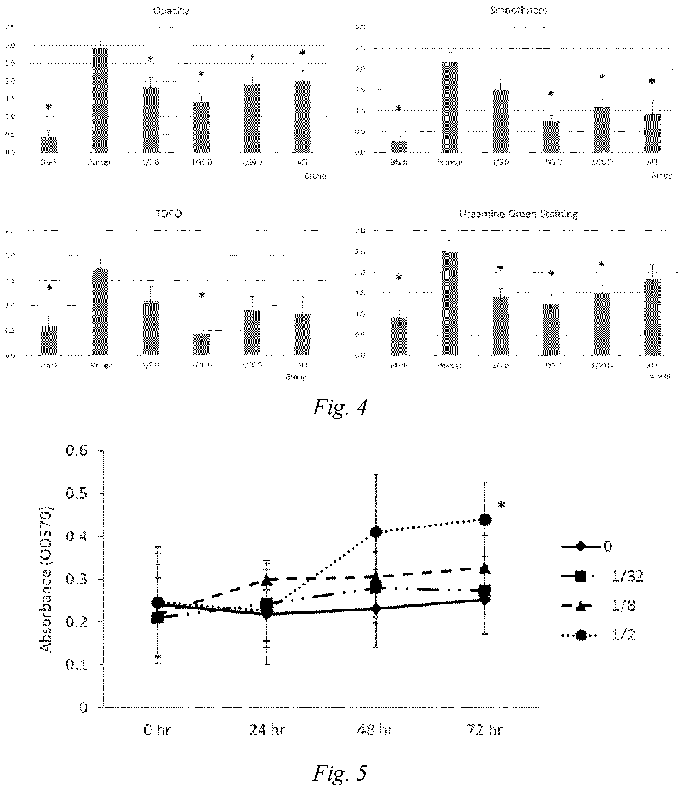

The corneal surface photography included assessments of cornea opacity, cornea smoothness, cornea topography, and Lissamine Green Stain examination, wherein the higher score of assessment represents the higher severity of damage. The result of photography was shown in Fig. 4.

It is shown in Fig. 4 that the damage of cornea in all of the groups treated with secretome of AFSC was mitigated. Specifically, the opacity of cornea of the groups treated with secretome of AFSC was lowered down, which also shows statistical significance (p<0.05). Moreover, the cornea smoothness, topography, and Lissamine Green Stain examination also show improvements with the treatment of secretome of AFSC.

Additionally, although AFT was used as a comparison herein and also shows improvement, the improvement with the treatment of AFT is inferior as shown in Fig. 4 and thus the result imply the secretome of AFSC is a potential substitute of AFT.

The eye tissue including cornea, lacrimal gland, and meibomian gland (also known as tarsal gland) were subject to tissue staining and IHC stain.

H&E stain showed the morphology of cell and indicates the integrity of tissue. The object of IHC includes Cox-2, p63, PCNA, NFκ-B, p53, and 4-HNE. Cox-2 and NFκ-B are the modulators of immune response and inflammation. P63 and PCNA are the modulators of cell proliferation and reproduction. p53 is an apoptosis factor. 4-HNE is correlated with oxidative stress.

Table 1 shows the quantitative result of H&E stain and IHC stain of Cox-2, p63, and PCNA in cornea tissue. (Mark "+" indicates the level of health.) As shown in Table 1, the treatment of secretome of AFSC is effective in relieving the inflammation response and enhancing the cell proliferation after damage of UVB in cornea tissue, wherein the expressions of p63 and PCNA are elevated but the expression of Cox-2 is reduced. The scores of the groups were evaluated below: Blank > 1/10 D > 1/5 D > 1/20 D > AFT > Damage.

Table 1

| Tissue | Stain | Blank | Damage | 1/5D | 1/10D | 1/20D | AFT |
|---|---|---|---|---|---|---|---|
| Cornea | H&E | ++++++ | + | ++++ | ++++++ | +++ | ++ |
| | Cox-2 | ++++++ | + | +++ | ++++++ | ++++ | ++ |
| | p63 | ++++++ | + | ++++ | ++++ | +++ | ++ |
| | PCNA | ++++++ | + | ++++ | ++++ | +++ | ++ |

Table 2 demonstrates the quantitative result of H&E stain and IHC stain of Cox-2, p53, and NFκ-B in lacrimal gland tissue. As shown in Table 2, the treatment of secretome of AFSC is effective in relieving the inflammation response and inhibiting apoptosis after damage of UVB in lacrimal gland tissue, wherein the expressions Cox-2, p53, and NFκ-B are reduced. The scores of the groups were evaluated below: Blank > 1/10 D > 1/5 D = 1/20 D > AFT > Damage.

Table 2

| Tissue | Stain | Blank | Damage | 1/5D | 1/10D | 1/20D | AFT |
|---|---|---|---|---|---|---|---|
| lacrimal gland | H&E | ++++++ | + | ++++ | ++++++ | +++ | ++ |
| | Cox-2 | ++++++ | + | +++ | ++++++ | ++++ | ++ |
| | NFκ-B | ++++++ | + | +++++ | ++++ | +++ | ++ |
| | p53 | ++++++ | + | +++++ | ++++ | +++ | ++ |

Table 3 demonstrates the quantitative result of H&E stain and IHC stain of Cox-2, 4-HNE, and NFκ-B in meibomian gland tissue. As shown in Table 3, the treatment of secretome of AFSC is effective in relieving the inflammation response and reducing oxidative stress after damage of UVB in meibomian gland tissue, wherein the expressions Cox-2, 4-HNE, and NFκ-B are reduced. The scores of the groups were evaluated below: Blank > 1/10 D > 1/5 D > 1/20 D > AFT > Damage.

Table 3

| Tissue | Stain | Blank | Damage | 1/5D | 1/10D | 1/20D | AFT |
|---|---|---|---|---|---|---|---|
| meibomian gland | H&E | ++++++ | + | ++++ | +++ | ++++++ | ++ |
| | Cox-2 | ++++++ | + | ++++ | +++++ | +++ | ++ |
| | NFκ-B | ++++++ | + | ++++ | +++++ | +++ | ++ |
| | 4-HNE | ++++++ | + | ++++ | +++++ | +++ | ++ |

In view of Table 1 to Table 3, the treatment of the secretome of AFSC was capable to maintain the integrity of cell tissue, relieve inflammation, and rescue cell from apoptosis and enhance proliferation after damage of UVB, suggesting that the secretome of AFSC should be a competent agent in restoring and recovering eye tissue such as cornea.

**Example 3**

In the present example, the human corneal epithelia cells were treated with the secretome of AFSC and the effects thereof were observed.

**Materials and Method**

The method obtaining the conditioned medium of AFSC is identical to that in Example 1 and does not repeated herein.

Human corneal epithelial cells (HCEC) (Thermo Fisher) were incubated in keratinocyte serum free medium (SFM) (Thermo Fisher) under the condition of 37°C and 5% $CO_2$.

In order to obtain the suspension of cells, the medium was discarded and the cells were washed with 3 ml DPBS (Dulbecco's phosphate buffered saline). 1ml 0.05% trypsin was added after DPBS was discarded and the cells were left under 37°C for 5-7 minutes.

After confirming the cells were round and detached under microscope, the cells were suspended in 2 ml medium to attenuate the reaction of trypsin.

20 μl suspension was taken out and the same volume of trypan blue was added and mixed with the suspension. Afterwards, 10 μl of the mixture was taken out and the cell number was counted on a cytometer.

A MTT assay was conducted to evaluate the effect of secretome of AFSC on cornea cells. The MTT assay is a colorimetric assay for assessing cell metabolic activity and viability. NAD(P)H-dependent cellular oxidoreductase enzymes may, under defined conditions, reflect the number of viable cells present. These enzymes are capable of reducing the tetrazolium dye MTT 3-(4,5-dimethylthiazol-2-yl)-2,5-diphenyltetrazolium bromide to its insoluble formazan, which has a purple color.

HCEC was disposed in 96-well plate with 8,000 cells in each well. 100 μl culture medium were added and the cells were incubated for 24 hours. Afterwards, the cells were treated with different concentrations of 100 μl solution of secretome of AFSC (1/2, 1/8, and 1/32 dilution) and the incubation was continued for 24, 48, and 72 hours separately.

When the incubation is ended according to the due time above, the medium was replaced with 200 μl MTT solution and the plate was disposed for 3 hours for reaction. The MTT solution was further replaced with DMSO to dissolve crystals. The cells were analyzed under ELISA reader to detect the absorbance under 570 nm.

On the other hand, the wound healing assay was conducted. $1.6 \times 10^5$ cells were incubated for 24 hours in a 12-well plate, each well of which contained 1 ml medium. When the incubation ended, 200 μl pipette tips were used to scratched 3 lines on the bottom of well. The medium was then discarded and the cells were washed with DPBS, followed by the addition of 500 μl medium. The plate was observed and photographed under 100X microscopy to record the starting status of cell migration (0 hour).

Subsquently, different concentrations (1/2, 1/8, and 1/32 dilution) of 500 μl solution of secretome of AFSC were added. The plate was further observed and photographed 6, 12, 24, and 48 hours after the starting of cell migration. The migrating distance was calculated according to the formula below:

$$Distance = [(D0-Dn)/D0] \times 100\%$$

wherein D0 is the width of scratch at 0 hour and Dn is the width of scratch after 6, 12, 24, and 48 hours.

All of the experiment result was presented as Mean ± SD (standard deviation). The statistics of homogeneity test was conducted by One way ANOVA and the significance at each time point was conducted by Scheffe's *post hoc* method. $p < 0.05$ indicates statistical significance; $p < 0.001$ indicates high statistical significance.

**Results**

The results of the MTT assay demonstrating the effects of the secretome of AFSC on human corneal cells are shown in Fig. 5, illustrating the correlation of treatment of different concentrations of secretome of AFSC and cell viability at 24, 48, and 72 hours.

As shown in Fig. 5, the absorbance of OD570 (cell viability) increased upon the levels of secretome of AFSC. The efficiency in improving viability also increased in accordance with the levels of secretome of AFSC. Amongst all the groups, the group treated with the 1/2 dilution of secretome of AFSC showed the most significant improvement.

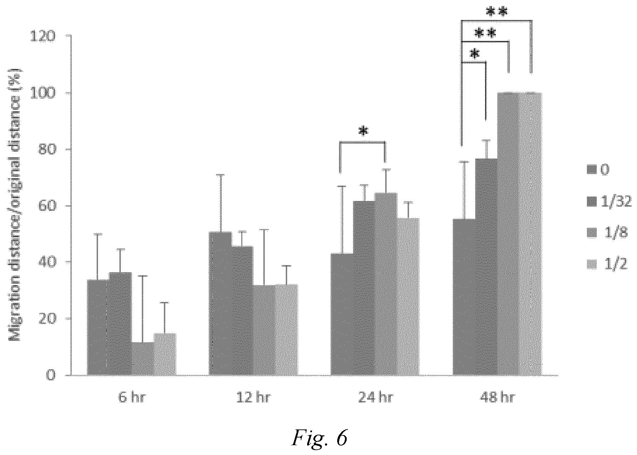

The results of the wound healing assay showing the effect of the secretome of AFSC on corneal cell migration are given in Fig. 6, illustrating the efficiency of migration upon the treatment of secretome of AFSC at 6, 12, 24, and 48 hours.

As shown in Fig. 6, the group treated without the secretome of AFSC (negative control) slightly and insignificantly migrated from 0 to 48 hours. On the contrary, the migration was obvious in the group treated with the secretome of AFSC from 24 to 48 hours. Furthermore, the groups treated with 1/2 and 1/8 dilution of the secretome of AFSC showed approximately 100% migration at 48 hours, indicating that the width of scratch was almost completely recovered and the group treated with 1/32 dilution of the secretome of AFSC showed approximately 80% migration.

Given the results of this example, the secretome of AFSC was capable of improving the survival and viability of human corneal cells as well as increasing the migrating efficiency of human corneal cell. Therefore, the secretome of AFSC shows potential therapeutic efficacy as an agent of dry eye.

**Claims**

1. A pharmaceutical composition for use in treating a dry eye disease, the pharmaceutical composition comprising a secretome of amniotic fluid stem cells;
   wherein the secretome is prepared by a method comprising the following steps: culturing amniotic fluid stem cells in a basal medium for 24-72 hours to obtain a culture medium, collecting a supernatant of the culture medium after centrifugation, and filtrating the supernatant to obtain the secretome.

2. The pharmaceutical composition for use according to claim 1, wherein the supernatant is filtrated by a filter having a pore size less than 0.5 $\mu$m.

3. The pharmaceutical composition for use according to claim 2, wherein the supernatant is filtrated by a filter having a pore size less than 0.22 $\mu$m.

4. The pharmaceutical composition for use according to claim 1, which is administered topically.

5. The pharmaceutical composition for use according to claim 4, which is in a form of eye gel, eye drop, eye bath solution, eye lotion, eye insert, eye ointment, or eye spray.

6. A pharmaceutical composition for use in corneal epithelia wound healing, the pharmaceutical composition comprising a secretome of amniotic fluid stem cells;
   wherein the secretome is prepared by a method comprising the following steps: culturing amniotic fluid stem cells in a basal medium for 24-72 hours to obtain a culture medium, collecting a supernatant of the culture medium after centrifugation, and filtrating the supernatant to obtain the secretome.

7. The pharmaceutical composition for use according to claim 6, wherein the supernatant is filtrated by a filter having a pore size less than 0.5 $\mu$m.

8. The pharmaceutical composition for use according to claim 7, wherein the supernatant is filtrated by a filter having a pore size less than 0.22 $\mu$m.

9. The pharmaceutical composition for use according to claim 6, which is administered topically.

10. The pharmaceutical composition for use according to claim 9, which is in a form of eye gel, eye drop, eye bath solution, eye lotion, eye insert, eye ointment, or eye spray.

**Patentansprüche**

1. Pharmazeutische Zusammensetzung zur Verwendung bei der Behandlung einer Erkrankung des trockenen Auges, worin die pharmazeutische Zusammensetzung ein Sekretom von Amnionflüssigkeits-Stammzellen umfasst; wobei das Sekretom durch ein Verfahren hergestellt wird, das die Schritte umfasst: Kultivieren von Amnionflüssigkeits-Stammzellen in einem Basismedium für 24-72 Stunden, um ein Kulturmedium zu erhalten, Sammeln eines Überstandes des Kulturmediums nach Zentrifugation und Filtrieren des Überstandes, um das Sekretom zu erhalten.

2. Pharmazeutische Zusammensetzung zur Verwendung nach Anspruch 1, wobei der Überstand durch einen Filter mit einer Porengröße von weniger als 0,5 $\mu$m filtriert wird.

3. Pharmazeutische Zusammensetzung zur Verwendung nach Anspruch 2, wobei der Überstand durch einen Filter mit einer Porengröße von weniger als 0,22 $\mu$m filtriert wird.

4. Pharmazeutische Zusammensetzung zur Verwendung nach Anspruch 1, welche topisch verabreicht wird.

5. Pharmazeutische Zusammensetzung zur Verwendung nach Anspruch 4, welche in Form eines Augengels, von Augentropfen, einer Augenbadlösung, einer Augenlotion, einer Augeneinlage, einer Augensalbe oder eines Augensprays vorliegt.

6. Pharmazeutische Zusammensetzung zur Verwendung bei der Wundheilung von Hornhautepithelien, worin die pharmazeutische Zusammensetzung ein Sekretom von Amnionflüssigkeits-Stammzellen umfasst; wobei das Sekretom durch ein Verfahren hergestellt wird, das die Schritte umfasst: Kultivieren von Amnionflüssigkeits-Stammzellen in einem Basismedium für 24-72 Stunden, um ein Kulturmedium zu erhalten, Sammeln eines Überstandes des Kulturmediums nach Zentrifugation und Filtrieren des Überstandes, um das Sekretom zu erhalten.

7. Pharmazeutische Zusammensetzung zur Verwendung nach Anspruch 6, wobei der Überstand durch einen Filter mit einer Porengröße von weniger als 0,5 $\mu$m filtriert wird.

8. Pharmazeutische Zusammensetzung zur Verwendung nach Anspruch 7, wobei der Überstand durch einen Filter mit einer Porengröße von weniger als 0,22 $\mu$m filtriert wird.

9. Pharmazeutische Zusammensetzung zur Verwendung nach Anspruch 6, welche topisch verabreicht wird.

10. Pharmazeutische Zusammensetzung zur Verwendung nach Anspruch 9, welche in Form eines Augengels, von Augentropfen, einer Augenbadlösung, einer Augenlotion, einer Augeneinlage, einer Augensalbe oder eines Augensprays vorliegt.

**Revendications**

1. Composition pharmaceutique pour utilisation dans le traitement d'une maladie oculaire sèche, la composition pharmaceutique comprenant un sécrétome de cellules souches du liquide amniotique; dans laquelle le sécrétome est préparé par une méthode comprenant les étapes suivantes: cultiver des cellules souches du liquide amniotique dans un milieu de base pendant 24 à 72 heures pour obtenir un milieu de culture, collecter un surnageant du milieu de culture après centrifugation, et filtrer le surnageant pour obtenir le sécrétome.

2. Composition pharmaceutique pour utilisation selon la revendication 1, dans laquelle le surnageant est filtré par un filtre ayant une taille de pore inférieure à 0,5 $\mu$m.

3. Composition pharmaceutique pour utilisation selon la revendication 2, dans laquelle le surnageant est filtré par un filtre ayant une taille de pore inférieure à 0,22 $\mu$m.

4. Composition pharmaceutique pour utilisation selon la revendication 1, qui est administrée par voie topique.

**5.** Composition pharmaceutique pour utilisation selon la revendication 4, qui se présente sous la forme d'un gel oculaire, d'une goutte oculaire, d'une solution pour bain oculaire, d'une lotion oculaire, d'un insert oculaire, d'un onguent oculaire, ou d'un spray oculaire.

**6.** Composition pharmaceutique pour utilisation dans la cicatrisation de l'épithélium cornéen, la composition pharmaceutique comprenant un sécrétome de cellules souches du liquide amniotique;
dans laquelle le sécrétome étant préparé par une méthode comprenant les étapes suivantes: cultiver des cellules souches du liquide amniotique dans un milieu de base pendant 24 à 72 heures pour obtenir un milieu de culture, collecter un surnageant du milieu de culture après centrifugation, et filtrer le surnageant pour obtenir le sécrétome.

**7.** Composition pharmaceutique pour utilisation selon la revendication 6, dans laquelle le surnageant est filtré par un filtre ayant une taille des pores est inférieure à 0,5 $\mu$m.

**8.** Composition pharmaceutique pour utilisation selon la revendication 7, dans laquelle le surnageant est filtré par un filtre ayant une taille de pore inférieure à 0,22 $\mu$m.

**9.** Composition pharmaceutique pour utilisation selon la revendication 6, qui est administrée par voie topique.

**10.** Composition pharmaceutique pour utilisation selon la revendication 9, qui se présente sous la forme d'un gel oculaire, d'une goutte oculaire, d'une solution pour bain oculaire, d'une lotion oculaire, d'un insert oculaire, d'un onguent oculaire ou d'un spray oculaire.

*Fig. 1*

*Fig. 2*

*Fig. 3*

*Fig. 4*

*Fig. 5*

*Fig. 6*

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2017100440 A1
- US 2008286378 A1